# EUROPEAN PATENT APPLICATION

(11) **EP 3 220 299 A1**
(43) Date of publication of application: **20.09.2017**
(21) Application number: 17161118.9
(22) Date of filing: 15.03.2017
(51) Int. Cl.: G06F 19/00

(54) **A METHOD FOR REMOTELY MONITORING AT LEAST ONE PATIENT**

(30) Priority: 16.03.2016 US 201662308866 P
(71) Applicant: CRF Inc., Plymouth Meeting, PA 19462 (US)
(72) Inventor: STROBRIDGE, Richard, Alpine, CA California 91901 (US)
(74) Representative: Berggren Oy, Helsinki & Oulu

(57) **Abstract**

A system and method is customizable to provide an individualized approach to healthcare. The system described herein has a wide range of customizable GUIs capable of adding or deleting a plurality of thresholds and parameters for at least one health monitoring device. The system and methodology of the present invention allow medical professionals to use a database of predetermined monitoring protocols to determine when to use a health monitoring device or take corrective action for a patient.

## Description

### Field of technology

The present invention relates generally to systems for remotely monitoring patients and more particularly to a system for remotely monitoring patients by providing customizable predetermined protocols to a patient to alert or trigger action by the patient in the use of a health monitoring device.

### Prior art

Individuals suffering from chronic conditions often require constant and consistent monitoring to manage their conditions. In order to provide the best possible healthcare for these patients, physicians need and employ a variety of health monitoring devices to collect physiological data. With the advent of health monitoring devices using wireless technologies, physicians are equipped to remotely monitor their patients while allowing the patient some semblance of a normal life.

There are a number of health monitoring devices with wireless capabilities currently available on the market including, but not limited to, glucose meters, chronic obstructive pulmonary disease (COPD) devices, thermometers, pulse oximeters, and heart monitors. The health monitoring devices use internal sensors to detect physiological changes that the doctor can use to recommend actions by the patient or another health monitoring device. In addition, the health monitoring devices may have predetermined thresholds that will trigger alerts if physiological conditions reach or exceed the predetermined threshold.

Within the context of remote monitoring, while the alerts based on predetermined thresholds are a valuable resource, there exists a need for a customizable system that takes into account a plurality of physiological measurements to provide an individualized protocol for the patient. Because patient's physiological conditions are constantly changing due to a plurality of factors including, but not limited to, environmental factors, types of food ingested, and presence of more than one acute or chronic conditions, in order to provide the best patient healthcare, a physician needs to be able to factor in as many physiological conditions as possible in a customizable, individualized system to effectively set contextually aware protocols.

### Short description

The present invention overcomes these and other deficiencies of the prior art by providing a system and method that is customizable to provide an individualized approach to healthcare. The system described herein has a wide range of customizable GUIs capable of adding or deleting a plurality of thresholds and parameters for at least one health monitoring device. The system and methodology of the present invention allow medical professionals to use a database of predetermined monitoring protocols to determine when to use a health monitoring device or take corrective action for a patient. Medical professionals are able to customize the preloaded protocols and create new monitoring protocols thereby creating an individualized approach to healthcare.

The foregoing, and other features and advantages of the invention, will be apparent from the following, more particular description of the preferred embodiments of the invention, the accompanying drawings, and the claims.

### List of figures

For a more complete understanding of the present invention, and the advantage thereof, reference is now made to the ensuing descriptions taken in connection with the accompanying drawings briefly described as follows:
Figs. 1A and 1B illustrate a graphical user interface (GUI) as presented to a physician within the system according to an embodiment of the invention;
Figs. 2A and 2B illustrate a GUI as used by the physician to customize individualized monitoring protocols for a patient using the system according to an embodiment of the invention;
Figs. 3A and 3B illustrate a GUI as presented to a patient to select symptoms and receive a physician recommended protocol from the system according to an embodiment of the present invention;
Fig. 4 illustrates an example of the data stored in the system from a patient profile according to an embodiment of the invention;
Fig. 5 illustrates an example of the data stored in the system from a patient's plurality of health monitoring device parameters and the customizable protocols predetermined by the physician according to an embodiment of the invention;
Fig. 6 illustrates a flow chart depicting the physician process of updating the patient's profile and associated protocols according to an embodiment of the invention;
Fig. 7 illustrates a flow chart depicting the process of monitoring the patient's data received from their health monitoring devices and executing appropriate protocols according to an embodiment of the invention; and
Fig. 8 illustrates a flow chart depicting the process of executing customized protocols for a patient according to an embodiment of the invention.

### Description of the invention

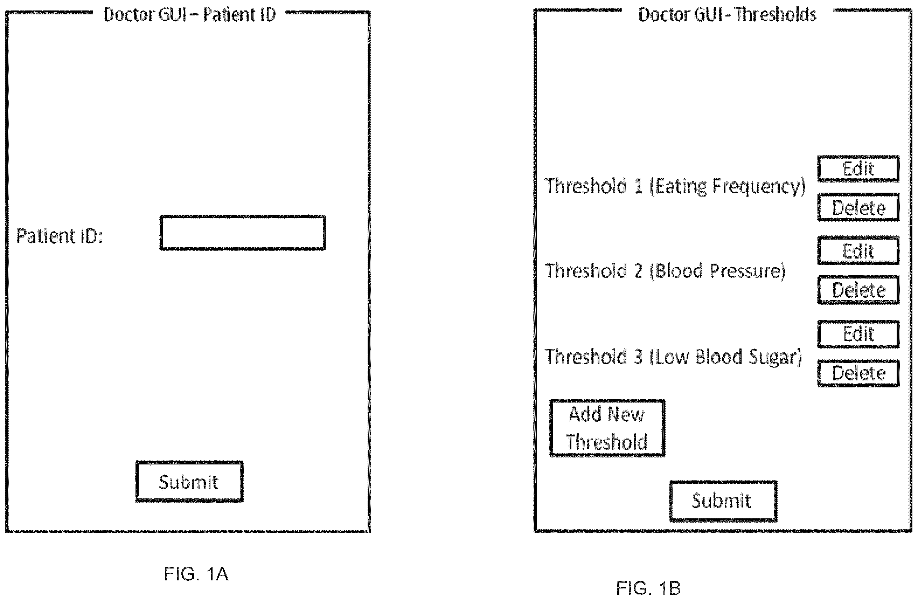

Further features and advantages of the invention, as well as the structure and operation of various embodiments of the invention, are described in detail below with reference to the accompanying Figs. 1-8.

The system described herein is characterized by the ability to customize various aspects of the system to provide an individualized approach to healthcare. In various embodiments, the system described herein has a wide range of customizable GUIs capable of adding or deleting a plurality of thresholds and parameters for at least one health monitoring device. Those of skill in the art will recognize that the plurality of thresholds and parameters for a particular patient varies with, for example, the acute and chronic conditions of that patient, environmental factors, tolerance of expense, the number and type of features desired, the amount and number of days of monitoring required, types of health monitoring devices being used, and the like.

While the system and methodology described herein is linked to the use of health monitoring devices generally, for ease and the sake of clarity, the figures described herein with respect to the system and method of the present invention will be described in the context of a patient using a glucose meter, food barcode scanner, and heart rate monitor. One of ordinary skill in the art will recognize, however, that any health monitoring device can be incorporated with the system and thresholds and parameters can be added and/or deleted to incorporate use of a wide range of health monitoring devices. In some embodiments, the health monitoring devices include, but are not limited to, spirometer, glucose meter, CPAP machine, indoor air quality (IAQ) meter, ventilator, pulse oximeter, sphygmomanometer, thermometer, nebulizers, heart monitors, and the like.

In a preferred embodiment of the present invention, the system communicates predetermined protocols to the patient via a health monitoring device or other connected device including, by way of non-limiting examples, smartphones, tablets, desktop computers, laptop computers, and the like.

The system and methodology of the present invention allow medical professionals to use a database of predetermined monitoring protocols to determine when to use a health monitoring device or take corrective action for a patient. Patient profiles are stored in a database on a network server, which medical professionals access through a GUI. The patient profile includes all the health monitoring devices associated with a particular patient according to the acute and chronic conditions of that patient. Certain protocols and thresholds are associated with the health monitoring devices of a particular patient. According to a preferred embodiment, medical professionals are able to customize the preloaded protocols and create new monitoring protocols. The monitoring protocols may have dependent steps and may prompt the patient to use a health monitoring device to collect health data and/or to take actions based on the collected data from the health monitoring device. The patient profile, including the customized thresholds and protocols, is communicated to the health monitoring device, or other connected device, of the patient. Sensor data from the health monitoring devices, calculated data, and input data is monitored by the system to determine if any thresholds are reached. If so, actions are taken and subsequent dependent monitoring steps may be executed.

Figs. 1A and 1B illustrate a GUI as presented to a physician within the system according to an embodiment of the invention. Those of skill in the art will appreciate that the GUIs described herein are only for illustration of the present invention and may be modified in whole or in part to facilitate the system and methodology according to the embodiments of the present invention. A physician accesses a database of patient profiles through a graphical user interface, Fig. 1A, by entering a unique identification associated with a particular patient. Fig. 1B, illustrates a representative GUI by which the physician may customize a plurality of thresholds individualized for that particular patient. For example, as illustrated in Fig. 1 B, the physician may set thresholds for eating frequency, blood pressure, and/or blood sugar.

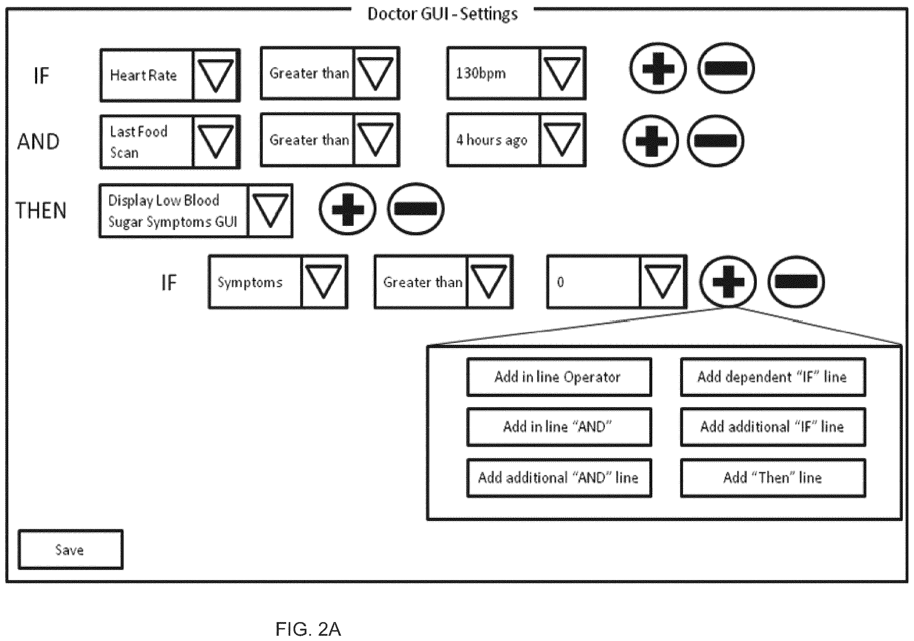

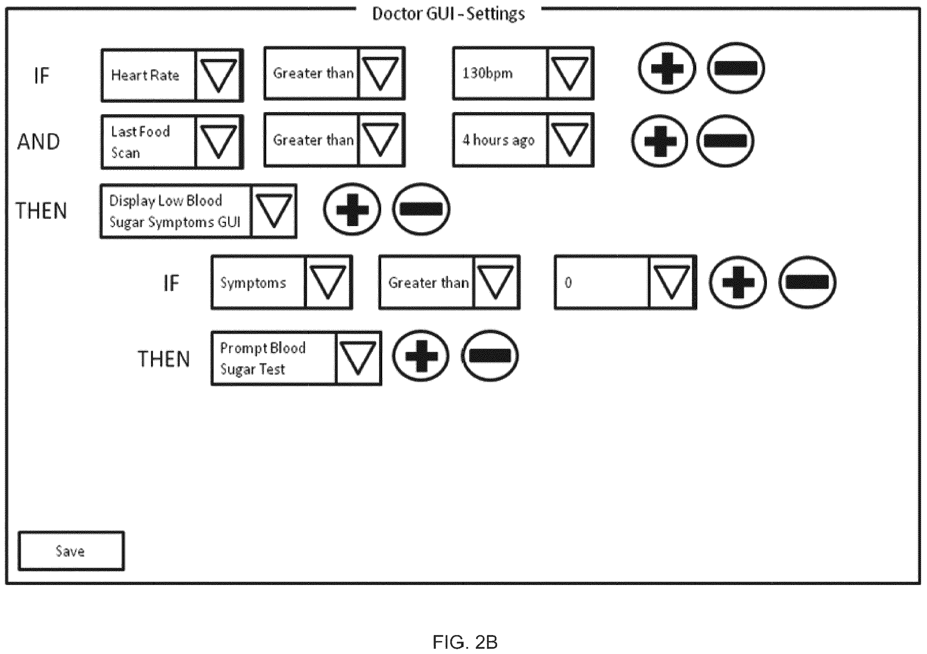

Figs. 2A and 2B illustrate a GUI as used by the physician to customize individualized monitoring protocols for a patient using the system according to an embodiment of the invention. After the physician accesses the patient profile and has customized the plurality of thresholds associated with that particular patient, the physician may customize individualized monitoring protocols based on the previously set thresholds as described with respect to Figs. 1A and 1B. In a preferred embodiment, the physician may customize the monitoring protocols by setting conditional statements. Fig. 2A illustrates a representative GUI with customized conditional statements for a particular patient. For example, in Fig. 2A, the physician has customized the conditional statements to display a set of low blood sugar symptoms to the patient via a connected device or health monitoring device. The physician is able to customize dependent conditional statements based on the symptoms selected by the patient in response to the displayed set of low blood sugar symptoms. Fig. 2B illustrates a representative GUI where the physician has customized dependent conditional statements based on the symptoms selected by the patient in response to the displayed set of low blood sugar symptoms. For example, if the patient selects certain symptoms of low blood sugar, then the patient may be prompted to use a glucose meter to test their blood sugar or take other corrective actions.

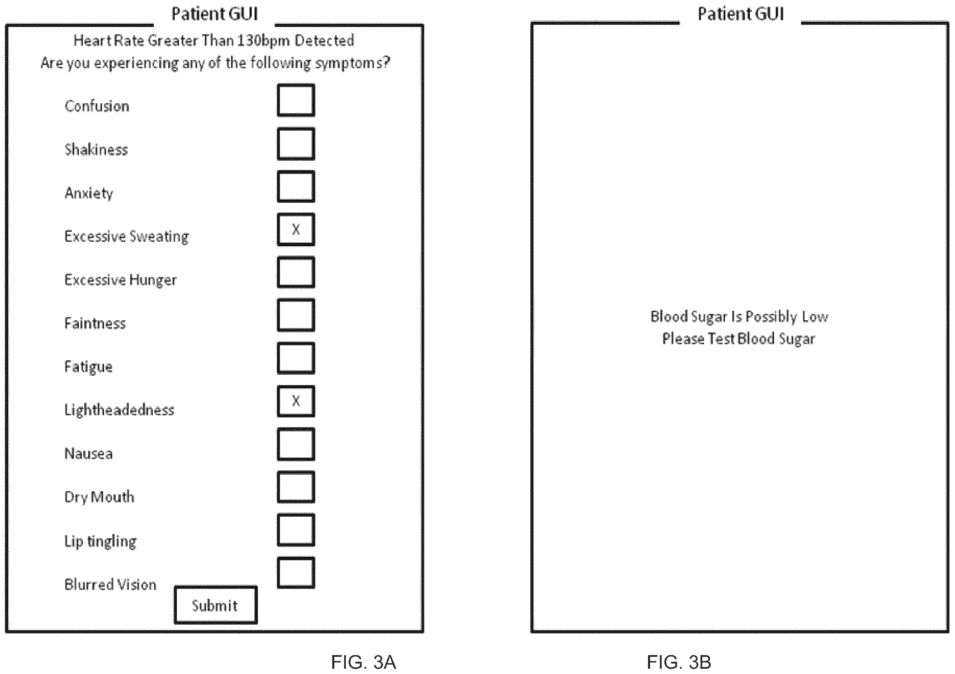

Figs. 3A and 3B illustrate a GUI as presented to a patient to select symptoms and receive a physician recommended protocol from the system according to an embodiment of the present invention. Fig. 3A illustrates a representative patient GUI where the physician has customized conditional statements to display a set of symptoms when the patient's heart rate is greater than 130 beats per minute (bpm). The patient is then able to select one or more symptoms that they may be experiencing due to the increased heart rate. In the representative GUI of Fig. 3A, the physician has customized this particular patient's profile to include a threshold for heart rate at 130 bpm. If the health monitoring device, in this case a heart rate monitor, detects a heart rate greater than 130 bpm, then the illustrative set of symptoms as depicted in Fig. 3A is displayed to the patient. In an alternative scenario, the patient GUI may display an action to be taken by the patient dependent upon the threshold set in the conditional statements. For example, Fig. 3B, depicts a representative action to be taken by a patient whose conditional statements suggest that their blood sugar levels may be low and the patient is required to test their blood sugar levels to determine further corrective actions if needed.

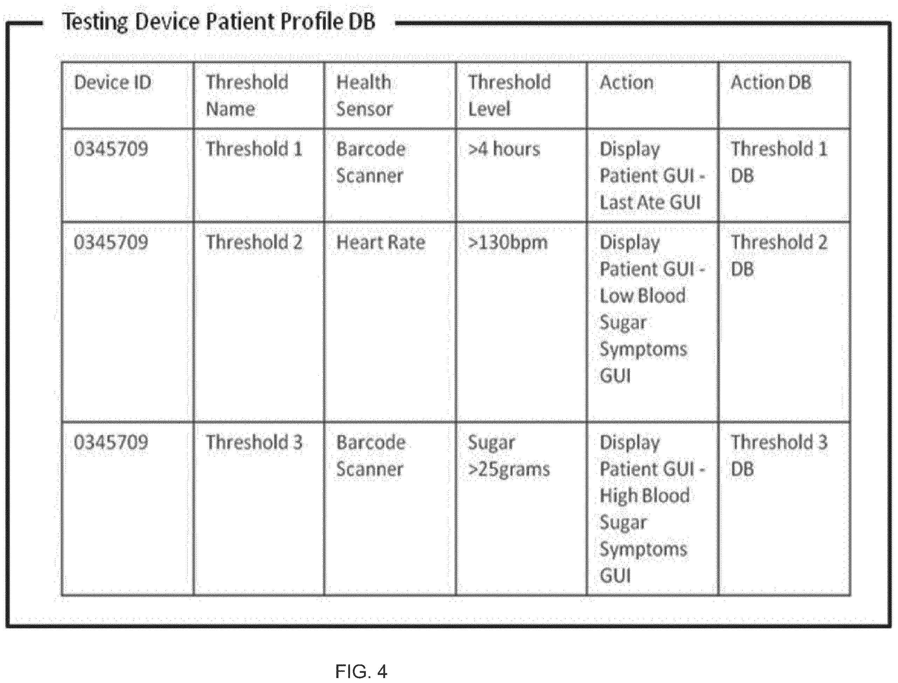

Fig. 4 illustrates an example of the data stored in the system from a patient profile according to an embodiment of the invention. In this example, the representative patient profile includes data from three (3) health monitoring devices: a food barcode scanner, heart rate monitor, and a glucose meter. Each device is identified in the patient profile database by a unique device ID that may be created automatically by the system when the health monitoring device is in communication with the system or be manually input by the physician or a system administrator. The patient profile database has customizable thresholds associated with each health monitoring device set by the physician. The physician may also customize actions to be taken dependent upon the customized thresholds for each health monitoring device. The actions may be associated with predetermined messages and alerts or sets of symptoms to be displayed to the patient. The predetermined messages and alerts or sets of symptoms to be displayed may be located in a separate database of the system that may be accessed when particular actions are triggered based upon the thresholds set for a particular health monitoring device in the patient's profile.

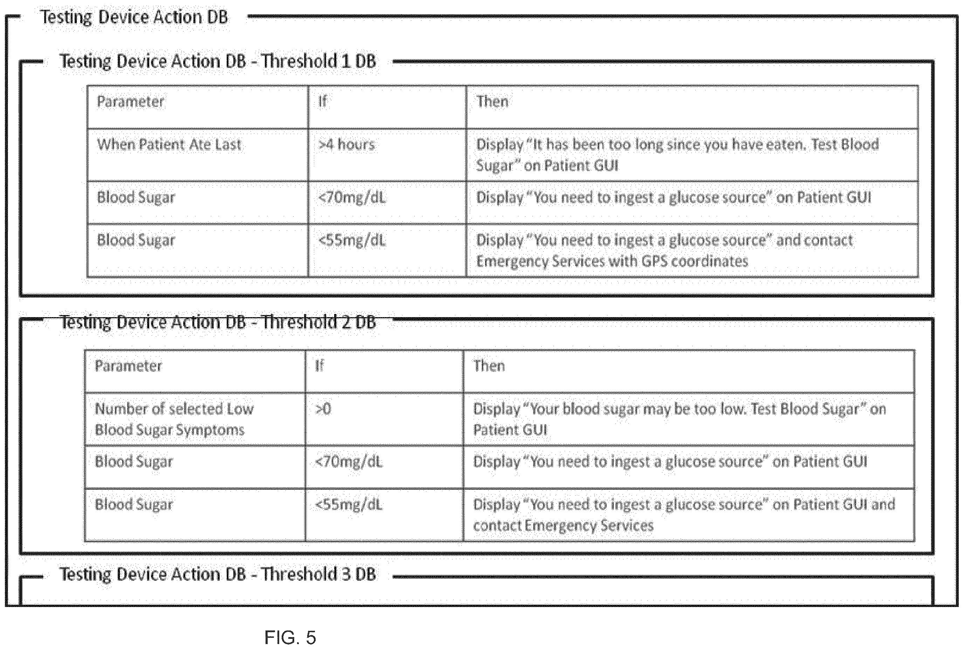

Fig. 5 illustrates an example of the data stored in the system from a patient's plurality of health monitoring device parameters and the customizable protocols predetermined by the physician according to an embodiment of the invention. The database depicted in Fig. 5, includes conditional statements associated with a particular patient's profile and health monitoring devices. For example, each conditional statement or set of conditional statements may be associated with one or more health monitoring devices such as a food bar scanner, heart rate monitor, and/or glucose meter. The conditional statements may include one or more dependent conditional statements. In addition, in some embodiments of the present invention, the conditional statement is associated with an action requiring the patient to select symptoms from a displayed set of symptoms associated with a threshold of a health monitoring device. Likewise, the conditional statement may be associated with a corrective action to be taken by the patient when a parameter is less than, equal to, or greater than a predetermined threshold of a health monitoring device. As an example illustrated in Fig. 5, if a patient last consumed food greater than four (4) hours ago as captured by a food barcode scanner and the patient's blood sugar levels as measured by a glucose meter are greater than 70 mg/dL, then the action to be taken by the patient is the ingestion of a glucose source. Alternatively, the same scenario may be set up as dependent conditional statements. For example, if the food bar scanner last captured data more than four (4) hours ago, then the patient may be prompted to test their blood sugar with a glucose meter. If the glucose meter test shows the patient's blood sugar level to be greater than 70 mg/dL, then the patient may be prompted to ingest a glucose source.

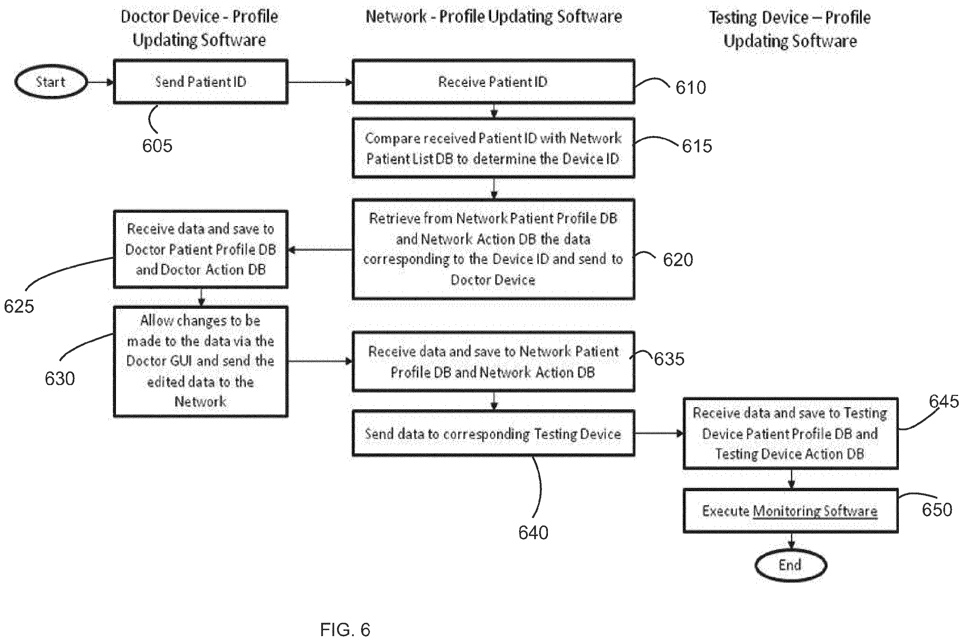

Fig. 6 illustrates a flow chart depicting the physician process of updating the patient's profile and associated protocols according to an embodiment of the invention. At 605, the physician enters a unique patient identification (ID) into the system. The network of the system receives the unique patient ID at 610 and compares the received patient ID with a database of the system to determine the health monitoring devices and associated unique device identifiers at 615. The system, at 620, retrieves the patient's profile and associated predetermined actions from system databases and sends to the physician's connected device(s). The physicians connected device may be, but is not limited to, smartphones, tablets, desktop computers, laptop computers, and the like. At 625, the physician's connected device(s) receives the patient's profile and associated predetermined actions. The customizability of the system, at 630, allows the physician to amend the data of the patient's profile and associated predetermined actions. The physician may then send the amended data, if needed, to the network of the system and save the data in the requisite database of the system at 635. The system may then, at 640, send the data from the patient's profile and associated predetermined actions to a patient's health monitoring device or other connected device. At 645, the patient's health monitoring device and/or other connected device receives and saves the data from the system. Depending upon the particular configuration of a particular patient's monitoring protocols, at 650, those monitoring protocols are executed. For example, dependent upon a patient's acute or chronic conditions, monitoring may be continuous or merely required at preset intervals throughout a given period.

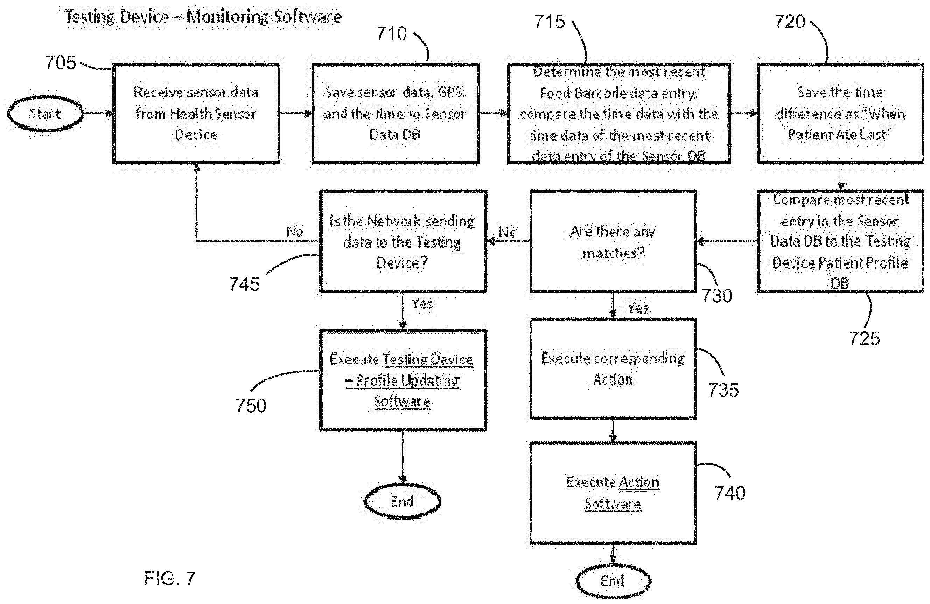

Fig. 7 illustrates a flow chart depicting the process of monitoring the patient's data received from their health monitoring devices and executing appropriate protocols according to an embodiment of the invention. Fig. 7 illustrates more detail to the process flow at the execution step at 650 of Fig. 6. As highlighted above, a particular monitoring configuration may dictate when and how often data is received, at 705, from one or more health monitoring devices linked to a patient's profile. The patient's health monitoring device and/or other connected device, at 710, saves the data received and time and location stamps the received data. The received data may be saved in a database of the health monitoring device and/or other connected device. At 715, the most recently received data is compared to the last saved entry in the database of the health monitoring device and/or other connected device. For example, in the context of a food barcode scanner, the system compares the most recently received data for the food barcode scanner and compares that data to the last entry saved to determine the time period since the patient last ate. After the comparison at 715, the system saves the difference, at 720, in the context of a food barcode scanner, as the time since the patient last ate. Those of skill in the art will recognize that steps 715 may not be necessary depending on the particular health monitoring device. For example, in the context of a glucose meter, step 715 is not necessary and the received data from the glucose meter is simply stored in the database.

At 725, the saved data from a particular health monitoring device is compared to the patient profile (as an example, see the database depicted in Fig. 4). At 730, the system determines whether there are matches for that particular health monitoring data in the patient profile. If there are matches in the patient profile, then at 735 and 740, the system executes the corresponding action and the action software respectively. If at 730, there are no matches in the patient profile for that particular data, then at 745, the system determines whether the system network is sending updated data to the health monitoring device and/or connected device. If updated data is being sent, then, at 750, the system executes the updating program as described in Fig. 6. If the updated data is not being sent, then the system starts the process flow over at step 705.

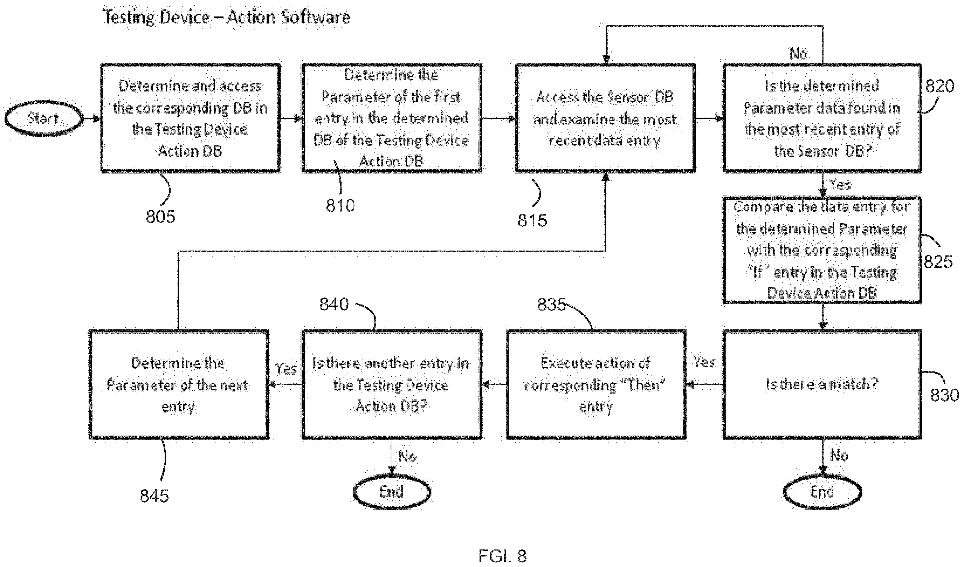

Fig. 8 illustrates a flow chart depicting the process of executing customized protocols for a patient according to an embodiment of the invention. Fig. 8 illustrates more detail to the process flow at the execution step at 740 of Fig. 7. At 805, the system determines and accesses the database corresponding to a health monitoring device of a patient (as an example, see the database depicted in Fig. 5). At 810, the system determines the first parameter and/or threshold of the health monitoring device. The most recent data entry for the health monitoring device being considered is determined at 815. The system, at 820, determines whether first parameter and/or threshold match the most recent data entry for that particular health monitoring device. If there is a match, then at 825 the system compares the "if" entry of the conditional statement with the most recent data entry from the corresponding health monitoring device. If the recent data entry satisfies the "if" condition of the conditional statement at 830, then the system executes the corresponding "then" action of the conditional statement at 835. If at 830 the condition is not satisfied, then the process ends. The system, after executing the action of the conditional statement, continues at 840 to determine whether there is another parameter and/or threshold or dependent parameter and/or threshold that require action. If there are no more parameters and/or thresholds, then the process ends. If, however, additional parameters and/or threshold require action, then the system, at 845, determines the next parameter and/or threshold and begins the process again at 815. Those of skill in the art will recognize that this process is capable of cycling through a plurality of iterations depending upon the number of customizable parameter and/or thresholds set by the physician.

Those of skill will in the art appreciate that the various illustrative logical blocks, modules, units, and algorithm steps described in connection with the embodiments disclosed herein can often be implemented as electronic hardware, computer software, or combinations of both. To clearly illustrate this interchangeability of hardware and software, various illustrative components, blocks, modules, and steps have been described above generally in terms of their functionality. Whether such functionality is implemented as hardware or software depends upon the particular constraints imposed on the overall system. Skilled persons can implement the described functionality in varying ways for each particular system, but such implementation decisions should not be interpreted as causing a departure from the scope of the invention. In addition, the grouping of functions within a unit, module, block, or step is for ease of description. Specific functions or steps can be moved from one unit, module, or block without departing from the invention.

The various illustrative logical blocks, units, steps and modules described in connection with the embodiments disclosed herein, and those provided in the accompanying documents, can be implemented or performed with a processor, such as a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein, and those provided in the accompanying documents. A general-purpose processor can be a microprocessor, but in the alternative, the processor can be any processor, controller, microcontroller, or state machine. A processor can also be implemented as a combination of computing devices, for example, a combination of a DSP and a microprocessor, a plurality of microprocessors, one or more microprocessors in conjunction with a DSP core, or any other such configuration.

The steps of a method or algorithm and the processes of a block or module described in connection with the embodiments disclosed herein, and those provided in the accompanying documents, can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM, or any other form of storage medium. An exemplary storage medium can be coupled to the processor such that the processor can read information from, and write information to, the storage medium. In the alternative, the storage medium can be integral to the processor. The processor and the storage medium can reside in an ASIC. Additionally, device, blocks, or modules that are described as coupled may be coupled via intermediary device, blocks, or modules. Similarly, a first device may be described a transmitting data to (or receiving from) a second device when there are intermediary devices that couple the first and second device and also when the first device is unaware of the ultimate destination of the data.

It is to be understood that the reference herein to a system, network, and/or database includes hardware and servers that may be physically located and internal to a particular facility or may be part of a cloud based system. One of skill in the art is capable of configuring such a system as described herein dependent upon the requirements of the facility employing the various embodiments of the present invention.

The above description of the disclosed embodiments, and that provided in the accompanying documents, is provided to enable any person skilled in the art to make or use the invention. Various modifications to these embodiments will be readily apparent to those skilled in the art, and the generic principles described herein, and in the accompanying documents, can be applied to other embodiments without departing from the spirit or scope of the invention. Thus, it is to be understood that the description and drawings presented herein, and presented in the accompanying documents, represent particular aspects and embodiments of the invention and are therefore representative examples of the subject matter that is broadly contemplated by the present invention. It is further understood that the scope of the present invention fully encompasses other embodiments that are, or may become, obvious to those skilled in the art and that the scope of the present invention is accordingly not limited by the descriptions presented herein, or by the descriptions presented in the accompanying documents.

## Claims

1. A method for remotely monitoring at least one patient in which method at least one health monitoring device is utilized for remote monitoring, **characterised in that** the method comprises steps
to provide an access to a medical professional to at least one database, which comprises patient specific profiles, the patient profiles comprising data of health monitoring devices associated with a particular patient and thresholds and protocols associated with these health monitoring devices,
to allow the medical professional to customize said thresholds and said protocols; to communicate the customized thresholds and protocols to the particular health monitoring devices or other connected devices.

2. A method according to Claim 1, **characterised in that** said protocols are arranged to inform for each patient when to use the health monitoring device/s and/or take action.

3. A method according to Claim 2, **characterised in that** the customization of the thresholds and the protocols is also arranged to create new protocols,

4. A method according to Claim 3, **characterised in that** the customization of the thresholds and the protocols is also arranged to add and/or delete thresholds or parameters for the health monitoring device/s

5. A method according to any of Claim 2 - 4, **characterised in that** the protocols have dependent steps.

6. A method according to any of Claim 2 or 5, **characterised in that** the customization of the thresholds is made first and after this the protocols are customized based on the customized thresholds.

7. A method according to any of Claim 1 - 6, **characterised in that** when customizing the protocols conditional statements for the particular patient are set.

8. A method according to any of Claim 1 - 7, **characterised in that** the health monitoring device or other connected device is identified in the patient profile by a unique device ID.

9. A method according to any of Claim 1 - 8, **characterised in that** the actions are associated with predetermined messages or sets of symptoms to be displayed to the patient.

10. A method according to any of Claim 1 - 9, **characterised in that** the access to the medical professional is provided through a graphical user interface.

11. A method according to any of Claim 1 - 9, **characterised in that** the method is implemented with at least one processor.

12. A system to remotely monitor at least one patient, which system comprises at least one health monitoring device for remote monitoring, **characterised in that** the system further comprises
at least one database, which comprises patient specific profiles, the patient profiles comprising data of health monitoring devices associated with a particular patient and thresholds and protocols associated with these health monitoring devices, and
at least one graphical user interface to provide an access to a medical professional to said at least one database,
which at least one health monitoring device, at least one database and at least one graphical user interface are arranged to be in communication, and which system is arranged to allow the medical professional to customize said thresholds and said protocols, and to communicate the customized thresholds and protocols to the particular health monitoring devices or other connected devices.

13. A system according to Claim 12, **characterised in that** said protocols are arranged to inform for each patient when to use the health monitoring device/s and/or take action.

14. A system according to Claim 13, **characterised in that** the customization of the thresholds and the protocols is also arranged to create new protocols,

15. A system according to Claim 14, **characterised in that** the customization of the thresholds and the protocols is also arranged to add and/or delete thresholds or parameters for the health monitoring device/s

16. A patient device being a health monitoring device, smartphone, tablet, desktop computer, laptop computer, and the like, **characterised in that** the patient device is arranged to be a part of a system according to any of claim 12 - 15, and to receive the customized protocols.

17. A practitioner interface being a graphical user interface, **characterised in that** the practitioner interface is arranged to be a part of a system according to claim 12.

18. A practitioner interface according to claim 17, **characterised in that** said practitioner interface is implemented as electronic hardware, computer software, or combinations of both.

19. A storage medium of software arranged to be with at least one processor, **characterised in that** the storage medium of the software is arranged for remotely monitoring at least one patient wherein at least one health monitoring device is utilized for remote monitoring, and the storage medium is arranged to provide steps
to provide an access to a medical professional to at least one database, which comprises patient specific profiles, each patient profile containing data of all health monitoring devices associated with a particular patient and thresholds and protocols associated with these health monitoring devices,
to allow the medical professional to customize thresholds and protocols;
to communicate the customized thresholds and protocols to the particular health monitoring devices or other connected device.

20. A storage medium according to Claim 19, **characterised in that** said protocols are arranged to inform for each patient when to use the health monitoring device/s and/or take action.

21. A storage medium according to Claim 20, **characterised in that** the customization of the thresholds and the protocols is also arranged to create new protocols,

22. A storage medium according to Claim 21, **characterised in that** the customization of the thresholds and the protocols is also arranged to add and/or delete thresholds or parameters for the health monitoring device/s

23. A storage medium according to any of Claim 20 - 22, **characterised in that** the protocols have dependent steps.

24. A storage medium according to any of Claim 20 or 23, **characterised in that** the customization of the thresholds is made first and after this the protocols are customized based on the customized thresholds.

25. A storage medium according to any of Claim 19 - 24, **characterised in that** when customizing the protocols conditional statements for the particular patient are set.

26. A storage medium according to any of Claim 19 - 25, **characterised in that** the health monitoring device or other connected device is identified in the patient profile by a unique device ID.

27. A storage medium according to any of Claim 19 - 26, **characterised in that** the actions are associated with predetermined messages or sets of symptoms to be displayed to the patient.

28. A storage medium according to any of Claim 19 - 27, **characterised in that** the access to the medical professional is provided through a graphical user interface.
